# EUROPEAN PATENT APPLICATION

(11) **EP 4 582 180 A1**
(43) Date of publication of application: **09.07.2025**
(21) Application number: 23859888.2
(22) Date of filing: 21.07.2023
(51) Int. Cl.: B01J 29/14, B01J 35/60, B01J 37/02, B01J 37/30, C07B 61/00, C07C 1/04, C07C 9/14

(54) **CATALYST, METHOD FOR MANUFACTURING SAME, AND METHOD FOR MANUFACTURING LIQUID FUEL**

(30) Priority: 02.09.2022 JP 2022139985
(71) Applicant: JFE Engineering Corporation, Chiyoda-ku, Tokyo 100-0011 (JP); National University Corporation University Of Toyama, Toyama-shi, Toyama 930-8555 (JP)
(72) Inventor: DOI, Makoto, Tokyo 100-0011 (JP); HASHIMOTO, Yukako, Tokyo 100-0011 (JP); URABE, Haruki, Tokyo 100-0011 (JP); IWASAKI, Toshihiko, Tokyo 100-0011 (JP); TSUBAKI, Noritatsu, Toyama-shi, Toyama 930-8555 (JP); GUO, Xiaoyu, Toyama-shi, Toyama 930-8555 (JP)
(74) Representative: Hoffmann Eitle
(86) International application number: PCT/JP2023/026834
(87) International publication number: WO 2024/048119

(57) **Abstract**

The purpose is to improve the yield of a hydrocarbon having a carbon number of 5 or more and produced using carbon monoxide and hydrogen as raw materials. A catalyst is capable of producing a hydrocarbon from a synthesis gas and includes a metallic catalyst containing a metal compound having activity in a Fischer-Tropsch synthesis reaction, the metallic catalyst being configured to produce the hydrocarbon from the synthesis gas, and a carrier catalyst containing zeolite supporting the metallic catalyst, the metal compound contains cobalt, and at least one metal selected from the group consisting of manganese and ruthenium, a supported amount of Mn is 1 to 3 wt%, a supported amount of Ru is 0.5 to 2 wt%, and a supported amount of Co is 10 to 30 wt%. The zeolite includes zeolite with pores which decomposes a carbon chain of the produced hydrocarbon, the pore is a mesopore having an opening diameter of 2 nm or more and 50 nm or less, and a ratio of silicon to aluminum in the zeolite is 2.5 or more and 3.5 or less.

## Description

### Field

The present invention relates to a catalyst, a method for producing the catalyst, and a method for producing liquid fuel, and is particularly suitable for application to a catalyst for producing a liquid fuel by reacting a mixed gas of carbon oxide and hydrogen, a method for producing the catalyst, and a method for producing a liquid fuel from carbon oxide using the catalyst. Background

In recent years, research and development for improving selectivity of an intended product by controlling a complicated reaction path using a synthesis technique based on a Fischer-Tropsch (FT) method in which a liquid hydrocarbon is synthesized from carbon monoxide (CO) and hydrogen (H₂) has been actively conducted. A technique in an oxide-based co-catalyst or a secondary metal co-catalyst is added alongside cobalt (Co), which is a conventional main catalyst, to improve the selectivity of the intended product has been proposed (see, for example, Patent Literatures 1 and 2).

In addition, a technique in which by using a modified meso-form zeolite cobalt catalyst, a liquid fuel is synthesized in one stage without secondary hydrogenation refining treatment from a synthesis gas that is a mixed gas of carbon monoxide and hydrogen has been proposed (see, for example, Non Patent Literature 1).

In Non Patent Literature 1 described above, a catalyst in which with respect to a Y-type zeolite carrier having mesopores, lanthanum (La) or potassium (K) is used as cations in the mesopores, and the supported amount of cobalt (Co) is 15 wt% is used as a catalyst. Such a catalyst has activity in a Fischer-Tropsch synthesis reaction, and is referred to as a FT synthesis catalyst for producing a hydrocarbon-based liquid fuel.

### Citation List

### Patent Literature

Patent Literature 1: Japanese Translation of PCT International Application Publication No. 2019-529065
Patent Literature 2: Japanese Patent Laid-Open No. 2015-131768

### Non Patent Literature

Non Patent Literature 1: "Integrated tuneable synthesis of liquid fuels via Fischer-Tropsch technology", Jie Li et al, Nature Catalysis volume 1, pages 787-793 (2018)

### Summary

### Technical Problem

When using the above-described FT synthesis catalyst, a liquid fuel containing a hydrocarbon having a carbon number of 5 to 20 is produced from a synthesis gas containing hydrogen (H₂) and carbon monoxide (CO), there is a problem that while the selectivity of the liquid fuel is high, the carbon monoxide conversion ratio (hereinafter, CO conversion ratio) is low. If the CO conversion ratio is low, the yield derived as a product of a conversion ratio and a selectivity decreases. Therefore, a highly active catalyst capable of improving the yield of a liquid product including a hydrocarbon having a carbon number of 5 or more has been desired.

The present invention has been made in view of the above, and an object of the present invention is to provide a catalyst capable of improving the yield of a hydrocarbon having a carbon number of 5 or more and 20 or less and produced using carbon monoxide and hydrogen as raw materials, a method for producing the catalyst, and a liquid fuel production method.

### Solution to Problem

(1) To solve the problem described above and to achieve the object, a catalyst according to one aspect of the present invention is capable of producing a hydrocarbon from a synthesis gas and includes: a metallic catalyst containing a metal compound having activity in a Fischer-Tropsch synthesis reaction, the metallic catalyst being configured to produce the hydrocarbon from the synthesis gas; and a carrier catalyst containing zeolite supporting the metallic catalyst, the metal compound containing cobalt, and at least one metal selected from the group consisting of manganese and ruthenium.
(2) In the present invention of the above-mentioned (1) of the catalyst according to one aspect of the present invention, a supported amount of the manganese is 1 wt% or more and 3 wt% or less.
(3) In the present invention of the above-mentioned (1) or (2) of the catalyst according to one aspect of the present invention, a supported amount of the ruthenium is 0.5 wt% or more and 2 wt% or less.
(4) In the present invention of any one of the above-mentioned (1) to (3) of the catalyst according to one aspect of the present invention, a supported amount of the cobalt is 10 wt% or more and 30 wt% or less.
(5) In the present invention of any one of the above-mentioned (1) to (4) of the catalyst according to one aspect of the present invention, the zeolite includes zeolite with pores which decomposes a carbon chain of the produced hydrocarbon, and each pore is a mesopore having an opening diameter of 2 nm or more and 50 nm or less.
(6) In the present invention of any one of the above-mentioned (1) to (5) of the catalyst according to one aspect of the present invention, a ratio of silicon to aluminum in the zeolite is 2.5 or more and 3.5 or less.
(7) A catalyst production method according to one aspect of the present invention is a catalyst production method for producing the catalyst according to the present invention of any one of the above-mentioned (1) to (6). The method includes: a pore forming step of forming mesopores in a carrier catalyst; and a catalyst supporting step of supporting a metal compound on the carrier catalyst, the catalyst supporting step including a step of supporting the metal compound containing cobalt and at least one of the metal compound containing manganese and the metal compound containing ruthenium on the carrier catalyst.
(8) In the present invention of the above-mentioned (7) of the catalyst production method according to one aspect of the present invention, the catalyst supporting step includes a melt impregnation step of melt-impregnating the carrier catalyst with the metal compound containing the cobalt and at least one of the metal compound containing the manganese and the metal compound containing the ruthenium.
(9) In the present invention of the above-mentioned (8) of the catalyst production method according to one aspect of the present invention, the melt impregnation step is a step of supporting the metal compound containing the cobalt on the carrier catalyst by a melt impregnation method, and then supporting at least one of the metal compound containing the manganese and the metal compound containing the ruthenium on the carrier catalyst by a melt impregnation method.
(10) In the present invention of the above-mentioned (8) of the catalyst production method according to one aspect of the present invention, the melt impregnation step involves supporting the metal compound containing cobalt along with at least one of the metal compounds containing manganese or ruthenium in parallel using the melt impregnation method.
(11) In the present invention of the above-mentioned (7) of the catalyst production method according to one aspect of the present invention, the catalyst supporting step includes an impregnation step of supporting the metal compound containing the cobalt on the carrier catalyst by an impregnation method, and then immersing the carrier catalyst supporting the cobalt in at least one of a solution containing the manganese and a solution containing the ruthenium to impregnate the carrier catalyst and the supported catalyst supported on the carrier catalyst with the at least one of the solution containing the manganese and the solution containing the ruthenium.
(12) In the present invention of the above-mentioned (7) of the catalyst production method according to one aspect of the present invention, the catalyst supporting step includes an impregnation step of supporting the metal compound containing the cobalt on the carrier catalyst by an impregnation method, and immersing the carrier catalyst supporting the cobalt in at least one of a solution containing the manganese and a solution containing the ruthenium to impregnate at least one of the carrier catalyst and the supported catalyst supported on the carrier catalyst with the at least one of the solution containing the manganese and the solution containing the ruthenium.
(13) In the present invention of the above-mentioned (7) of the catalyst production method according to one aspect of the present invention, the catalyst supporting step includes: a melt impregnation step of melt-impregnating the carrier catalyst with the metal compound containing the cobalt; and an impregnation step of immersing the carrier catalyst supporting the metal compound containing the cobalt, which is obtained in the melt impregnation step, in at least one of a solution containing the manganese and a solution containing the ruthenium to impregnate at least one of the carrier catalyst and the supported catalyst supported on the carrier catalyst with the at least one of the solution containing the manganese and the solution containing the ruthenium.
(14) In the present invention of any one of the above-mentioned (7) to (13) of the catalyst production method according to one aspect of the present invention, in the carrier catalyst, a cation is either pre-coordinated or introduced through a cation-exchange step using an ion-exchange method, which is performed prior to the catalyst supporting step.
(15) In the present invention of the above-mentioned (14) of the catalyst production method according to one aspect of the present invention, the cation is at least one cation selected from the group consisting of lanthanum, potassium, lithium, sodium and cerium.
(16) A liquid fuel production method according to one aspect of the present invention includes producing a liquid fuel including a hydrocarbon from a synthesis gas by a Fischer-Tropsch synthesis method using the catalyst according to the present invention of any one of the above-mentioned (1) to (6).

### Advantageous Effects of Invention

The catalyst, the method for producing the catalyst, and the liquid fuel production method according to the present invention enables improvement of the yield of a hydrocarbon having a carbon number of 5 or more and 20 or less, the hydrocarbon being produced using carbon monoxide and hydrogen as raw materials.

### Brief Description of Drawings

FIG. 1 is a flowchart for illustrating a catalyst production method for producing a catalyst according to an embodiment of the present invention.
FIG. 2 is a flowchart for illustrating catalyst supporting treatment in the catalyst production method shown in FIG. 1.
FIG. 3 is a graph illustrating a hydrocarbon distribution for each catalyst in a produced hydrocarbon, for illustrating an effect of addition of manganese in a catalyst according to an embodiment of the present invention.
FIG. 4 is a graph illustrating a hydrocarbon distribution for each catalyst in a produced hydrocarbon, for illustrating an effect of addition of manganese in a catalyst according to an embodiment of the present invention.
FIG. 5 is a graph illustrating a CO conversion ratio, a selectivity and a yield with respect to a supported amount of manganese in a hydrocarbon produced using a catalyst according to an embodiment of the present invention.
FIG. 6 is a graph illustrating a CO conversion ratio, a selectivity and a yield with respect to a supported amount of manganese in a hydrocarbon produced using a catalyst according to an embodiment of the present invention.
FIG. 7 is a graph illustrating a hydrocarbon distribution for each catalyst in a produced hydrocarbon, for illustrating an effect of addition of ruthenium in a catalyst according to an embodiment of the present invention.
FIG. 8 is a graph illustrating a hydrocarbon distribution for each catalyst in a produced hydrocarbon, for illustrating an effect of addition of ruthenium in a catalyst according to an embodiment of the present invention.
FIG. 9 is a graph illustrating a CO conversion ratio, a selectivity and a yield with respect to a supported amount of ruthenium in a hydrocarbon produced using a catalyst according to an embodiment of the present invention.
FIG. 10 is a graph illustrating a CO conversion ratio and a yield of C8-C16 in a produced hydrocarbon, and a proportion of waxes C16+, for each Si/Al ratio of a catalyst according to an embodiment of the present invention.

### Description of Embodiments

Hereinafter, an embodiment of the present invention will be described with reference to the drawings. In addition, the present invention is not limited by an embodiment described below. First, in describing an embodiment of the present invention, experiments and intensive studies conducted by the present inventor for solving the above-described problems will be described in order to facilitate understanding of the present invention.

First, the present inventor has conducted studies on problems concerning a catalyst which is used as a conventional FT (Fischer-Tropsch) synthesis catalyst and in which with Y-type mesoporous zeolite as a carrier catalyst, cobalt (Co) as a metal catalyst is supported in cation-exchanged mesopores (a Co-supported FT synthesis catalyst). Note that the mesopores are pores having an opening diameter of 2 nm or more and 50 nm or less, and have a peak in the range of 10 nm or more and 20 nm or less.

That is, according to the findings by the present inventor, when the above-described Co-supported Y-type mesoporous zeolite catalyst is used, and the supported amount (also referred to as a catalyst amount) of cobalt (Co) is increased for improving the yield in production of a liquid fuel including a hydrocarbon (CₙH₂ₙ₊₂) having a carbon number n of 5 to 20, the mesopores may be blocked, which is not preferable. Therefore, the present inventor has conducted studies on various metal catalysts other than Co, and arrived at the addition of a highly active catalyst. According to the studies by the present inventor, it is desirable to add manganese (Mn) as a metal catalyst for improving the yield of a liquid fuel including a hydrocarbon having a carbon number of 5 to 20 by adding a highly active metal catalyst. In other words, the present inventors have arrived at the use of a compound containing Co and Mn, which is a metal compound having activity in a Fischer-Tropsch synthesis reaction, as a metallic catalyst for producing a hydrocarbon from a synthesis gas containing carbon (C) and hydrogen (H₂). In addition, it has been found that among the carrier catalysts that support Co and Mn, the zeolite studied is preferable, and in particular, Y-type zeolite having mesopores is preferable. Further, from the experiments conducted by the present inventor, it has been found that the supported amount of Mn is preferably 1 wt% or more and 3 wt%, more preferably 1.5 wt% or more and 2.5 wt% or less, and the optimum value is preferably about 2.0 wt%.

The present inventor has produced a liquid fuel using the above Co-supported FT synthesis catalyst to which Mn is added (a MnCo-supported FT synthesis catalyst), and the present inventor has found that the CO conversion ratio is not significantly improved. Therefore, the present inventor has further conducted studies, conducted various experiments and intensive studies on a metal catalyst capable of improving the CO conversion ratio, and devised a method in which ruthenium (Ru) is added to a Co-supported FT synthesis catalyst. The present inventor has conducted an experiment of producing a hydrocarbon from a synthesis gas using a Co-supported FT synthesis catalyst supporting Ru (a RuCo-supported FT synthesis catalyst), and confirmed that the production of the hydrocarbon is improved over the entire carbon number range, leading to improvement of the CO conversion ratio. In addition, it has been found that among the carrier catalysts supporting both Co and Ru, the zeolite under study is the preferred choice, with Y-type zeolite containing mesopores being particularly advantageous. Further, from the experiment conducted by the present inventor, it has been found that the supported amount of Ru is preferably 0.5 wt% or more and 2.0 wt%, more preferably 0.5 wt% or more and 1.5 wt% or less, and the optimum value is preferably about 1.0 wt%.

The present inventor has further advanced the above studies, and conducted studies on a Co-supported FT synthesis catalyst (RuMnCo-supported FT synthesis catalyst) obtained by adding Mn and Ru in combination, and has diligently conducted studies on conditions for maximizing the yield, and the effects thereof. The present inventor has produced a liquid fuel from a synthesis gas using a RuMnCo-supported FT synthesis catalyst, and confirmed considerable improvement of the yield of the liquid fuel. On the other hand, the present inventor has found that under the conditions for maximizing the yield of a hydrocarbon (maximum yield conditions), a wax including a hydrocarbon having a high carbon number of more than 20 is produced, covers the FT synthesis catalyst, and is deposited thereon, and the CO conversion ratio decreases with the lapse of time.

The present inventor has also conducted studies on reduction of the amount of wax, and has devised increasing the amount of a hydrocarbon with a low carbon number, which has a small carbon number n, for reducing the amount of wax produced. The present inventor has conducted studies on a method for increasing the amount of a hydrocarbon having a low carbon number. Additionally, a method has been developed to adjust the silicon-to-aluminum (Si/Al) ratio in zeolite, thereby enhancing the acid sites on the surface layer. This strengthening improves the cracking efficiency, facilitating the decomposition of carbon chains and shifting the carbon number distribution toward lower carbon number. This enables suppression of production of wax in the production of hydrocarbon. The present inventor has conducted experiments with attention paid to the amount of wax produced, and it has been found that the Si/Al ratio of zeolite, as an average over the zeolite, is typically 2.5 or more and 3.5 or less, preferably 2.7 or more and 3.1 or less, and more preferably 2.84 or more and 3.03 or less, and the optimum value is preferably about 2.94.

The present inventor has found, on the basis of the above studies, that cobalt and at least one metal selected from the group consisting of manganese and ruthenium are preferably contained as a metal compound supported on the FT synthesis catalyst. In addition, the present inventor has devised adjusting the hydrocarbon distribution by adjusting the Si/Al ratio of zeolite within the range of 2.5 to 3.5. The present invention has been devised on the basis of the above intensive studies by the present inventor.

### (Examples of carrier catalyst)

First, the carrier catalyst used for the FT synthesis catalyst according to the present embodiment is used as a hydrogenation catalyst, and includes zeolite, that is, aluminosilicate, and preferably Y-type zeolite in the present embodiment. When zeolite is provided on the surface layer, activated carbon, silicon carbide, silicon dioxide, titanium dioxide, zirconium dioxide, magnesium oxide, zinc oxide or a mixture thereof may be contained as the support, an intermediate material between the support and the zeolite, and a binder material.

### (Method for producing catalyst)

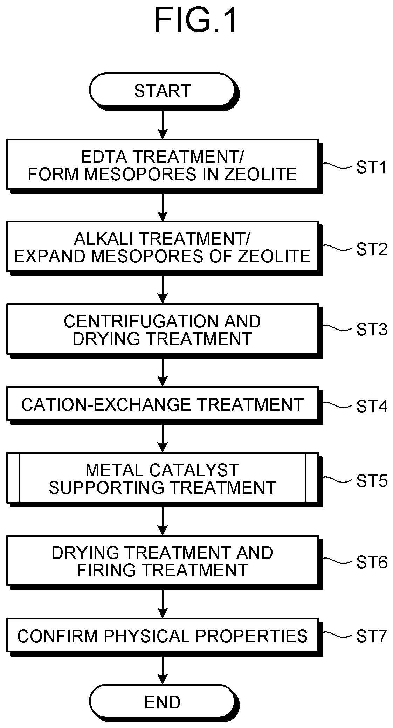

Next, a method for producing a FT synthesis catalyst that is a catalyst according to an embodiment, which has been devised by the above-described intensive studies, will be described. FIG. 1 is a flowchart for illustrating a method for producing a FT synthesis catalyst according to an embodiment.

As shown in FIG. 1, in the method for producing a catalyst according to an embodiment, first, microporous zeolite powder made from Y-type zeolite (manufactured by Tosoh Corporation) is provided as a raw material. Here, Y-type zeolite which is, for example, 6.7 g in mass and has a Si/Al ratio of 2.8 and in which sodium (Na) ions are coordinated in the molecular backbone is used. Note that as the zeolite, L-type zeolite can also be used.

In Step ST1, EDTA treatment is performed on the Y-type zeolite to form mesopores in the zeolite (pore forming step). In addition, the Si/Al ratio of the Y-type zeolite can be increased by eliminating Al from the Y-type zeolite. Specifically, in Step ST1, the zeolite powder 6.7 g in mass is mixed with an EDTA (ethylenediaminetetraacetic acid) aqueous solution with a concentration of 0.07 mol/L and a volume of 100 ml. Next, in a vessel such as a flask, the mixture is refluxed and stirred at a temperature of 100°C (373 K) for six hours. Note that adjustment of the time of EDTA treatment enables adjustment of the amount of elimination of Al, so that the Si/Al ratio of the zeolite can be adjusted. Thereafter, the solid powder is filtered and dried with, for example, an air dryer at 120°C for 12 hours.

Next, the process proceeds to Step ST2, where alkali treatment, for example, sodium hydroxide (NaOH) treatment is performed on the Y-type zeolite in which mesopores are formed, thereby expanding the mesopores (pore expanding step). In addition, the Si/Al ratio of the Y-type zeolite can be decreased by eliminating Si from the Y-type zeolite. Specifically, for example, in Step ST2, the solid powder of the Y-type zeolite is mixed with a NaOH aqueous solution with a concentration of 0.4 mol/L and a volume of 50 ml. Next, the mixture is stirred, for example, at a temperature of 61°C (338 K) for 30 min (0.5 hours).

Thereafter, the process proceeds to Step ST3, where powder is separated in about five minutes using, for example, a centrifuge at a rotation speed of 9000 rpm, and drying treatment is performed for 12 hours using, for example, an air dryer at 120°C. By this, a Y-type mesoporous zeolite (hereinafter, Yₘₑₛₒ) carrier is obtained.

Next, the process proceeds to Step ST4, where by an ion-exchange method, cation-exchange treatment using, for example, lanthanum (La) as a cation is performed on the Y-type mesoporous zeolite carrier in which sodium (Na) is coordinated as a cation in advance (cation-exchange step). Note that as the cation, not only La but also potassium (K), lithium (Li), cerium (Ce) or the like can be used. Specifically, for example, in Step ST4, a Y-type mesoporous zeolite carrier 1.0 g in mass is mixed with a lanthanum nitrate solution with a concentration of 0.2 mol/L and a volume of 100 ml. Next, the mixture is stirred at a temperature of 80°C (353 K) for 12 hours to perform cation-exchange treatment. Thereafter, the mixture is centrifuged to separate powder, and subjected to firing treatment in an air atmospheric pressure atmosphere at a temperature of 550°C (823 K) for six hours to produce powder of a Y-type mesoporous zeolite La (hereinafter, Yₘₑₛₒ-La) carrier.

The cation exchange amount is adjusted within the range of 0.1% or more and 10% or less depending on temperature and time conditions, and is typically about 1%. In addition, in the present embodiment, a cation-exchange step where zeolite in which Na is coordinated as a cation in advance is used is described, but the present invention is not limited to a case where a cation is coordinated in advance, and a cation may be coordinated to zeolite in which a cation has not been coordinated.

Next, the process proceeds to Step ST5, where metal catalyst supporting treatment is performed on the produced Yₘₑₛₒ-La carrier by, for example, a melt impregnation method (catalyst supporting step). Specifically, for example, cobalt nitrate (Co(NO₃)₂·6H₂O), at least one of manganese nitrate (Mn(NO₃)₂) and ruthenium nitrate (Ru(NO₃)₃), and powder of a Yₘₑₛₒ-La carrier are kneaded for, for example, 30 minutes (0.5 hours). Thereafter, for example, a vessel such as a glass bottle is charged with the kneaded product, and sealed, followed by melt impregnation at a temperature of 50°C (333 K) for 48 hours (melt impregnation step). By this, the Yₘₑₛₒ-La carrier is impregnated with a metal compound of Co and at least one of Mn and Ru in the mesopores. Note that there may be cases where Co is exposed from the mesopores due to shallowness of the mesopores or support on the surfaces of the mesopores. In this case, Mn or Ru may be supported on the surface or the periphery of Co.

Here, the supported amount of Co is, for example, 15 wt%, the supported amount of Mn is, for example, 2 wt%, and the supported amount of Ru is, for example, 1 wt%. The supported metal compound having activity in a FT synthesis reaction is not always reduced by 100% ultimately, but when it is assumed to be reduced by 100%, the supported amount is a ratio of the mass of metal in the metal compound having activity in the FT synthesis reaction to the total mass of the catalyst (the total mass of the catalyst that produces a hydrocarbon from a synthesis gas).

After the metal catalyst supporting treatment in Step ST5, the process proceeds to Step ST6, where drying treatment and firing treatment are performed. As the firing treatment, for example, nitrogen (N₂) gas is circulated at a temperature of 400°C (673 K) and a flow rate of 40 mL/min for four hours, thereby eliminating nitrogen (N) to form cobalt oxide, manganese oxide or ruthenium oxide. By this, a MnCo-supported FT synthesis catalyst (Mn-Co/Yₘₑₛₒ-La catalyst), a RuCo-supported FT synthesis catalyst (Ru-Co/Yₘₑₛₒ-La catalyst), or a RuMnCoFT synthesis catalyst (Ru-Mn-Co/Yₘₑₛₒ-La catalyst) is obtained. Note that when potassium (K) is used as a cation in the ion-exchange treatment in Step ST4, a MnCo-supported FT synthesis catalyst (Mn-Co/Yₘₑₛₒ-K catalyst), a RuCo-supported FT synthesis catalyst (Ru-Co/Yₘₑₛₒ-K catalyst), a RuMnCoFT synthesis catalyst (Ru-Mn-Co/Yₘₑₛₒ-K catalyst), or the like is obtained. In addition, when another cation such as cerium (Ce) is used as the cation, the moiety of "-La" or "-K" in each FT synthesis catalyst is replaced with "-Ce" or the other cation.

Thereafter, if necessary, the process proceeds to Step ST7, where the physical properties of the product are confirmed by, for example, X-ray diffraction or gas chromatography to confirm the physical properties of the FT synthesis catalyst. Specifically, for evaluating the reactivity of the catalyst, for example, a tubular reactor is filled with the produced FT synthesis catalyst, and hydrogen gas at a temperature of 400°C is circulated to perform reduction treatment. Thereafter, under conditions of a temperature of 250°C and a pressure of 2.0 MPa, the synthesis gas is brought into contact with the FT synthesis catalyst by adjusting the flow rate so that the catalyst mass W to the synthesis gas flow rate F (W/F) is, for example, 10 gh/mol. Note that the ratio of hydrogen (H₂) to carbon monoxide (CO) in the synthesis gas is set to 1 to 2 (H₂/CO = 1 to 2). The compositions of the synthetic gas supplied and the gas discharged from the outlet of the tubular reactor are analyzed using methods such as gas chromatography (GC). This allows for the measurement of key parameters, including the CO conversion ratio, CH₄ selectivity, selectivity for hydrocarbons with a carbon number of five or greater (C₅⁺ selectivity), and the productivity of liquid hydrocarbons.

### (Modification of metal catalyst supporting treatment method)

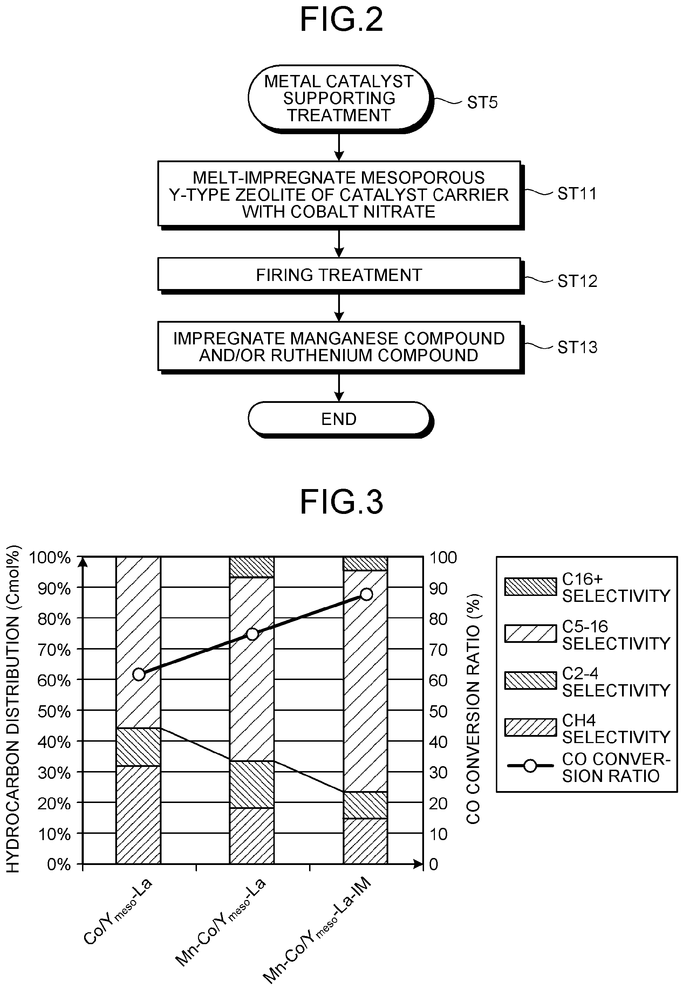

Next, an impregnation method (Impregnation: IM method), which is a modification of the above-described catalyst carrying treatment in Step ST5, will be described. FIG. 2 is a flowchart illustrating a metal catalyst supporting treatment method according to a modification.

That is, in Step ST11, the Yₘₑₛₒ-La carrier powder produced in Step ST4 shown in FIG. 1 is melt-impregnated with cobalt nitrate (Co(NO₃)₂·6H₂O) as a metal compound by the above-described melt impregnation method. Note that an impregnation method may be carried out instead of the melt impregnation method.

Thereafter, the process proceeds to Step ST12, where firing treatment is performed. By this, a Co-supported FT synthesis catalyst (Co/Yₘₑₛₒ-La catalyst) is obtained. Note that the supported amount of Co is, for example, 15 wt%. Next, the process proceeds to Step ST13, where the CO/Yₘₑₛₒ-La catalyst powder obtained by the firing treatment is immersed in at least one of a manganese nitrate (Mn (NO₃)₂) solution and a ruthenium nitrate (Ru(NO₃)₃) solution, thereby being impregnated. By this, the carrier catalyst having mesopores and the supported catalyst supported on the carrier catalyst are impregnated with at least one of Mn and Ru corresponding to the solution. Note that the melt impregnation method and the impregnation method may be carried out in parallel.

Thereafter, the process proceeds to Step ST6, where as the firing treatment, for example, nitrogen (N₂) gas is circulated at a temperature of 400°C (673 K) and a flow rate of 40 mL/min for four hours, thereby eliminating nitrogen (N) to form cobalt oxide, manganese oxide or ruthenium oxide. By the above, a MnCo-supported FT synthesis catalyst (Mn-Co/Yₘₑₛₒ-La catalyst), a RuCo-supported FT synthesis catalyst (Ru-Co/Yₘₑₛₒ-La catalyst), or a RuMnCoFT synthesis catalyst (Ru-Mn-Co/Yₘₑₛₒ-La catalyst) is obtained. Note that these catalysts may be collectively referred to as a FT synthesis catalyst.

### (Method for producing hydrocarbon)

Next, a method for producing a hydrocarbon using a FT synthesis catalyst produced as described above, in particular, a method for producing a liquid fuel such as jet fuel will be described. The method for producing a hydrocarbon according to the present embodiment is the same as a conventional method for producing a liquid fuel except that the above-described Y-type mesoporous zeolite catalyst supporting Co and at least one of Mn and Ru is used as a FT synthesis catalyst.

That is, first, hydrogen (H₂) gas and carbon monoxide (CO) gas are produced from methane (CH₄), water (H₂O), oxygen (O₂) and carbon dioxide (CO₂) by a synthesis gas production step. Next, a hydrocarbon (CₙH₂ₙ₊₂) is produced from the synthesis gases by a Fischer-Tropsch synthesis method (also referred to as a FT method or a Fischer-Tropsch synthesis reaction method) using the above-described FT synthesis catalyst.

Subsequently, various liquid hydrocarbons are separated and refined into lower olefins (carbon number n = 2 to 4: C2-C4), naphtha (carbon number n = 5 to 10: C5-C10), kerosene (carbon number n = 10 to 14: C10-C14), light oil (carbon number n = 14 to 20: C14-C20), wax (carbon number n > 20: C20+) and the like by an upgrading step. Note that as the jet fuel, a hydrocarbon having a carbon number n of 8 to 16 (C8-C16) is mainly used. In addition, as the liquid fuel, a hydrocarbon having a carbon number n of 5 to 20 (C5-C20) is mainly used. By the above, a liquid fuel containing a liquid hydrocarbon is produced by a FT method using a FT synthesis catalyst.

### (Effects of addition of Mn to FT synthesis catalyst)

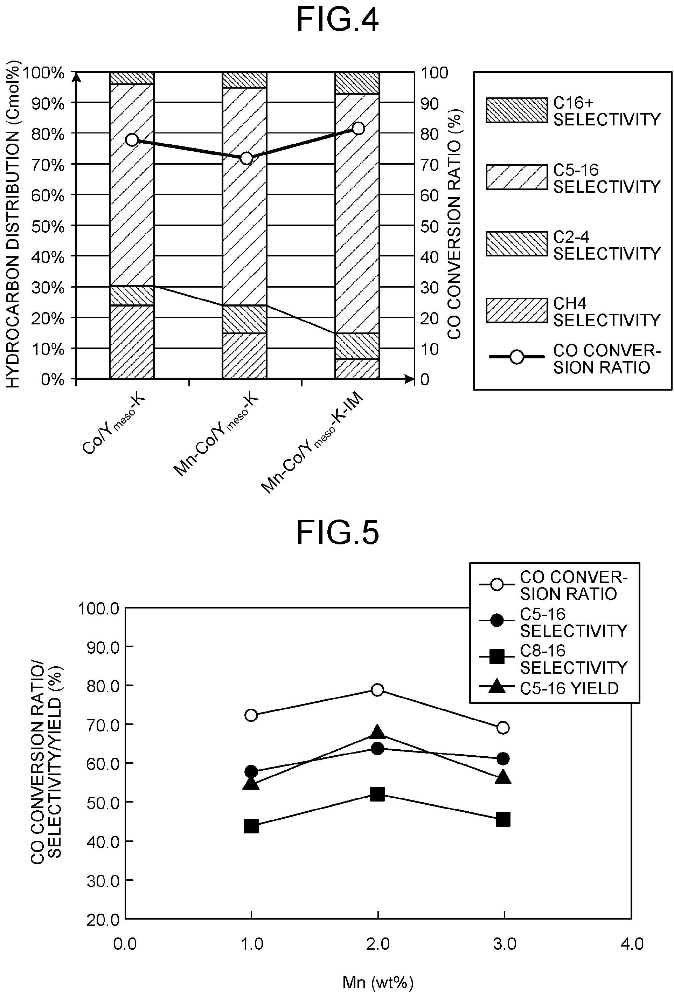

Next, effects of the FT synthesis catalyst produced as described above will be described. FIGS. 3 and 4 are graphs illustrating a hydrocarbon distribution for each catalyst in a produced hydrocarbon, for illustrating effects of addition of manganese (Mn) in the catalyst according to the present embodiment. Note that the hydrocarbon distribution in FIGS. 3 and 4 shows a distribution when a hydrocarbon is produced from a synthesis gas having a H₂/CO ratio of 2.0 (H₂/CO = 2.0). In addition, in the graph shown in FIGS. 3 and 4, "Co/Yₘₑₛₒ-La" indicates a Co-supported Y-type mesoporous zeolite La catalyst which is a FT synthesis catalyst prepared by ion-exchange with La as a cation, referred to as a Co/Yₘₑₛₒ-La catalyst. Similarly, "Mn-Co/Yₘₑₛₒ-La" indicates a MnCo-supported Y-type mesoporous zeolite La catalyst which is a FT synthesis catalyst further supporting Mn. Further, "Mn-Co/Yₘₑₛₒ-La-IM" indicates a FT synthesis catalyst impregnated with Mn and Co to support Mn and Co by the IM method shown in FIG. 2. The same applies to other graphs.

First, "Co/Yₘₑₛₒ-La" in FIG. 3 and "Co/Yₘₑₛₒ-K" in FIG. 4 are FT synthesis catalysts as conventional techniques which are described in Non Patent Literature 1. In the conventional techniques, as described in Non Patent Literature 1, the selectivity is as high as 72 to 86%, but the CO conversion ratio is extremely low and 30 to 40% in a liquid fuel including a hydrocarbon having a carbon number of 5 to 20, under the condition of a H₂/CO ratio of 1.0 (H₂/CO = 1.0). When synthesis is performed under the condition of a H₂/CO ratio of 2.0 (H₂/CO = 2.0) for increasing the CO conversion ratio, as shown in FIG. 3, the selectivity is 55% in a liquid fuel including a hydrocarbon having a carbon number of 5 to 16 and a CO conversion ratio of 62% in the case of "Co/Yₘₑₛₒ-La". In addition, as shown in FIG. 4, the selectivity is 66% in a liquid fuel including a hydrocarbon having a carbon number of 5 to 16 and a CO conversion ratio of 78% in the case of "Co/Yₘₑₛₒ-K".

On the other hand, from "Mn-Co/Yₘₑₛₒ-La" and "Mn-Co/Yₘₑₛₒ-La-IM" shown in FIG. 3, it can be seen that in the embodiment described above, a considerable improvement is made with the selectivity being 60 to 71% and the CO conversion ratio being 75 to 88% in a liquid fuel including a hydrocarbon having a carbon number of 5 to 20, under the condition of a H₂/CO ratio of 2.0 (H₂/CO = 2.0). In addition, from "Mn-Co/Yₘₑₛₒ-K" and "Mn-Co/Yₘₑₛₒ-K-IM" shown in FIG. 4, it can be seen that under the condition of a H₂/CO ratio of 2.0 (H₂/CO = 2.0), the CO conversion ratio is 72 to 82% and does not significantly change, but the selectivity is improved to 71 to 78% in a liquid fuel having a carbon number of 5 to 20.

Therefore, when the FT synthesis catalyst described in Non Patent Literature 1 is used, both the CO conversion ratio and the selectivity of a liquid fuel including a hydrocarbon having a carbon number of 5 to 16 are low under the condition of a H₂/CO ratio of 2.0 (H₂/CO = 2.0). On the other hand, it can be seen from FIGS. 3 and 4 that in the embodiment described above, addition of Mn by about 2% can increase the selectivity by 5 to 16 points in a liquid fuel having a carbon number of 5 to 20, and leads to an increase by 11 to 29 points in yield obtained by a product of the selectivity and the CO conversion ratio (selectivity × CO conversion ratio).

In addition, it can be seen from FIG. 3 that in the FT synthesis catalyst on which a metal catalyst is supported by the IM method (see FIG. 2), the selectivity of hydrocarbons having a carbon number n of 5 or more (C5+) increases from 60% to 71% as compared to the FT synthesis catalyst on which a metal catalyst is supported by the melt impregnation method. Similarly, it can be seen that the CO conversion is improved from 75% to 88%. In addition, it can be seen from FIG. 4 that in the FT synthesis catalyst on which a metal catalyst is supported by the IM method, the selectivity of hydrocarbons having a carbon number n of 5 or more (C5+) increases from 71% to 78% as compared to the FT synthesis catalyst on which a metal catalyst is supported by the melt impregnation method. That is, it can be seen from FIGS. 3 and 4 that in the FT synthesis catalyst, the selectivity of a liquid fuel (C5+) and the CO conversion can be improved by supporting Mn by the IM method.

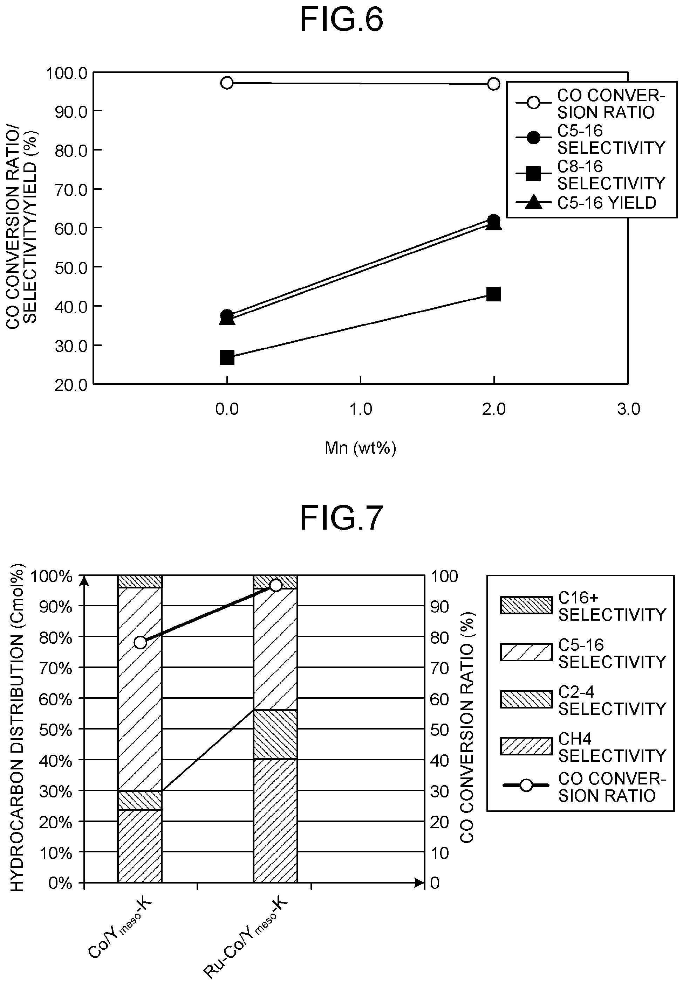

FIGS. 5 and 6 are graphs illustrating a CO conversion ratio, a selectivity and a yield with respect to a supported amount of manganese in a hydrocarbon produced using a catalyst according to the present embodiment. FIG. 5 is a graph when a synthesis gas having a H₂/CO ratio of 1.5 (H₂/CO = 1.0) is used, and FIG. 6 is a graph when a synthesis gas having a H₂/CO ratio of 2.0 (H₂/CO = 1.0) is used.

From FIG. 6, it can be seen that by adding Mn as a metal catalyst to the FT synthesis catalyst, the selectivity of hydrocarbons having carbon numbers n of 5 to 16 (C5-C16) and 8 to 16 (C8-C16), that is, a liquid fuel suitable for a jet fuel is improved without decreasing the CO conversion ratio. Since the selectivity of hydrocarbons having carbon numbers n of 5 to 16 and 8 to 16 is improved without decreasing the CO conversion ratio, the yields of hydrocarbons having carbon numbers n of 5 to 16 and 8 to 16 can be improved.

In addition, it can be seen from FIGS. 5 and 6 that when the supported amount of Mn supported on the FT synthesis catalyst is more than 0 wt% and 3 wt% or less, the CO conversion ratio, as well as the selectivity and the yield of hydrocarbons with carbon numbers n of 5 to 16 and 8 to 16, can be improved. Further, from FIG. 5, it can be seen that the supported amount of Mn is preferably around 2 wt%, for example, preferably 1 wt% or more and 3 wt% or less, and more preferably 1.5 to 2.5 wt%.

### (Effects of addition of Ru to FT synthesis catalyst)

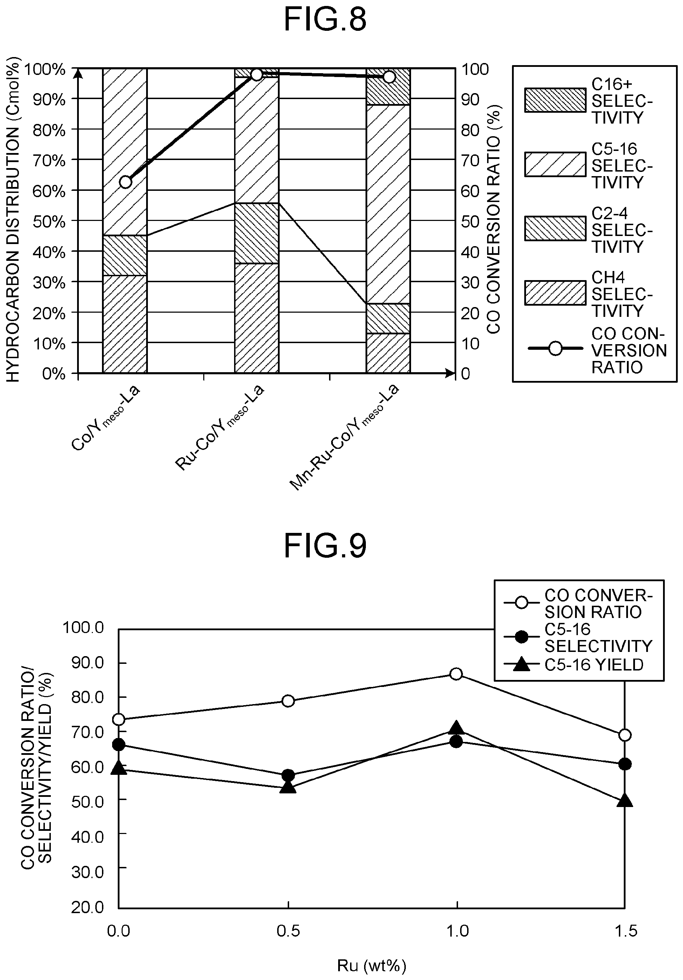

FIGS. 7 and 8 are graphs illustrating a hydrocarbon distribution for each catalyst in a produced hydrocarbon, for illustrating an effect of addition of ruthenium in a catalyst according to the present embodiment. As the synthesis gas, a synthesis gas having a H₂/CO ratio of 2.0 (H₂/CO = 2.0) is used. From FIGS. 7 and 8, it can be seen that in the present embodiment, addition of Ru to the FT synthesis catalyst (Co/Yₘₑₛₒ-K catalyst) as a conventional technique can increase the CO conversion ratio to substantially 100% in a hydrocarbon having a carbon number n of 5 to 16 (C5-C16), that is a liquid fuel.

Further, from FIG. 8, it can be seen that by using a FT synthesis catalyst in which Mn and Ru are both added and supported, the selectivity of a liquid fuel having a carbon number n of 5 to 16 can be improved without decreasing the CO conversion ratio from substantially a little under 100%, as compared to a FT synthesis catalyst supporting only Ru. That is, it can be seen that the Ru-Mn-Co/Yₘₑₛₒ-La catalyst according to the present embodiment can improve the yield of a liquid fuel having a carbon number of 5 to 16.

In addition, FIG. 9 is a graph illustrating a CO conversion ratio, a selectivity and a yield with respect to a supported amount of ruthenium in a hydrocarbon produced using a catalyst according to the present embodiment. FIG. 9 is a graph when a synthesis gas having a H₂/CO ratio of 1.5 (H₂/CO = 1.5) is used.

From FIG. 9, it can be seen that when Ru as a metal catalyst is increased to a supported amount of 0.5 wt%, 1.0 wt% and 1.5 wt% in the FT synthesis catalyst, the CO conversion ratio can be improved up to a supported amount of about 1.0 wt%. In addition, it can be seen that the CO conversion ratio can be maintained at a sufficient value up to a supported amount of 1.5 wt%. That is, it can be seen that the supported amount of Ru is preferably 0.5 wt% or more and 1.5 wt% or less, and more preferably around 1.0 wt%.

### (Suppression of production of wax)

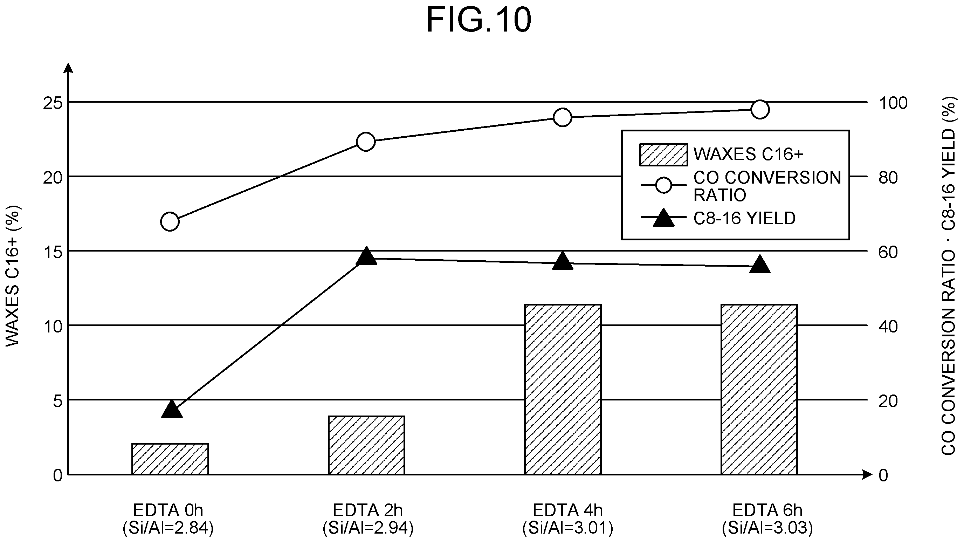

FIG. 10 is a graph illustrating a CO conversion ratio in a produced hydrocarbon and a yield of a liquid fuel having a carbon number n of 5 to 16 (C5-C16), and a proportion of waxes C16+ which are a hydrocarbon containing wax having a carbon number of more than 16 as a main component, for each Si/Al ratio of the catalyst according to the present embodiment.

From FIG. 10, it can be seen that in the FT synthesis catalyst, adjustment of the Si/Al ratio of the zeolite within the range of 2.5 or more and 3.5 or less (Si/Al ratio = 2.5 to 3.5), preferably 2.7 or more and 3.1 or less (Si/Al ratio = 2.7 to 3.1), and more preferably 2.84 or more and 3.03 or less (Si/Al ratio = 2.84 to 3.03) by changing the time of EDTA treatment to zero to six hours can control production of wax. The Si/Al ratio can be adjusted by the time of the EDTA treatment described above. From FIG. 10, in particular, it can be seen that when the time of EDTA treatment is two hours and the Si/Al ratio is about 2.94, it is possible to set the yield and the CO conversion ratio to high values while suppressing production of wax.

The embodiment described above has the following advantages over Patent Literature 1 and 2. That is, in the technique described in Patent Literature 1, a cobalt-based catalyst and a silica (SiO₂) catalyst carrier are used, and in the technique described in Patent Literature 2, a catalyst containing at least one metal selected from the group of ruthenium (Ru), cobalt (Co), and iron (Fe) or a compound and a β-type zeolite carrier having a Si/Al ratio of 13 or more are used. On the other hand, the embodiment described above is different in that the carrier is zeolite of Y type, L type or the like with a Si/Al ratio of 10 or less in which a cobalt-based catalyst is used. In addition, the catalyst according to the embodiment described above can improve reactivity and selectivity as compared to the catalysts described in Patent Literature 1 and 2.

According to the embodiment described above, it is possible to improve the yield of a hydrocarbon having a carbon number of 5 or more and 20 or less (C5-C20), preferably a hydrocarbon having a carbon number of 8 or more and 16 or less (C8-C16), which is produced using carbon monoxide (CO) and hydrogen (H₂) as raw materials, that is a jet fuel.

From an industrial point of view, it is possible to provide a catalyst capable of directly producing a liquid fuel such as jet fuel from carbon monoxide and hydrogen, and a technique for producing a liquid fuel can be provided efficiently and inexpensively. The catalyst according to the embodiment described above has been confirmed to be superior in catalyst performance to conventional techniques.

The embodiments of the present invention have been described in detail above, the present invention is not limited to the embodiments described above, and it is possible to make various modifications based on the technical idea of the present invention. For example, the numerical values given in the embodiments described above are merely examples, and numerical values different from those given above may be used as necessary. The present invention also includes components formed by appropriately combining those of the embodiments and modifications described above.

### (Modifications of metal compound supporting treatment method)

The method for supporting a metal compound having activity in a FT synthesis reaction on a catalyst carrier is not limited to the selection and treatment method of the above-described melt impregnation method and impregnation method, and it is also possible to perform the support only by melt impregnation or only by the impregnation method by optimizing the conditions and method for production for mass production and reduction of production cost, or to use a method in which Co, Mn and Ru are supported by one treatment.

That is, the catalyst supporting step may include a melt impregnation step of melt-impregnating the carrier catalyst with a metal compound containing cobalt and at least one of a metal compound containing manganese and a metal compound containing ruthenium. The melt impregnation step may be a step of supporting a metal compound containing cobalt on a carrier catalyst by a melt impregnation method, and then supporting at least one of a metal compound containing manganese and a metal compound containing ruthenium by a melt impregnation method. Alternatively, the melt impregnation step may be a step of supporting a metal compound containing cobalt and at least one of a metal compound containing manganese and a metal compound containing ruthenium on a carrier catalyst substantially in parallel by a melt impregnation method.

Further, the catalyst supporting step may include an impregnation step of supporting a metal compound containing cobalt on a carrier catalyst by an impregnation method, and then immersing the carrier catalyst supporting cobalt in at least one of a solution containing manganese and a solution containing ruthenium, thereby impregnating the carrier catalyst and the supported catalyst supported on the carrier catalyst. Alternatively, the catalyst supporting step may include an impregnation step of supporting a metal compound containing cobalt on a carrier catalyst by an impregnation method, and immersing the carrier catalyst supporting cobalt in at least one of a solution containing manganese and a solution containing ruthenium, thereby impregnating at least one of the carrier catalyst and the supported catalyst supported on the carrier catalyst. Here, the term "supported catalyst" refers to catalysts such as Co, Mn and Ru supported on the carrier catalyst.

### (Supported amount of Co)

The supported amount of the metal compound having activity in the FT synthesis reaction on the catalyst carrier is 5 to 50 mass%. For example, when cobalt (Co) is used, the content is preferably 10 to 30 wt%, and more preferably 15 wt%. If the supported amount is in the range of less than 10 wt%, the FT synthesis activity cannot be sufficiently exhibited, and if the supported amount is in the range of more than 30 wt%, the zeolite pores are blocked, so that the efficiency in utilization of supported Co decreases.

In addition, further effects and modifications can be easily derived by those skilled in the art. The broader aspects of the invention are not limited to the specific details and representative embodiments presented and described above. Accordingly, various changes can be made without departing from the spirit or scope of the general inventive concept which is defined by the appended claims and their equivalents.

### Industrial Applicability

The catalyst, the method for producing the catalyst, and the method for producing a liquid fuel according to the present invention are suitable for application to a catalyst for producing a liquid fuel by reacting a mixed gas of carbon oxide and hydrogen, a method for producing the catalyst, and a method for producing a liquid fuel from carbon oxide using the catalyst.

## Claims

1. A catalyst which is capable of producing a hydrocarbon from a synthesis gas, the catalyst comprising:
a metallic catalyst containing a metal compound having activity in a Fischer-Tropsch synthesis reaction, the metallic catalyst being configured to produce the hydrocarbon from the synthesis gas; and
a carrier catalyst containing zeolite supporting the metallic catalyst,
the metal compound containing cobalt, and at least one metal selected from the group consisting of manganese and ruthenium.

2. The catalyst according to claim 1, wherein
a supported amount of the manganese is 1 wt% or more and 3 wt% or less.

3. The catalyst according to claim 1, wherein
a supported amount of the ruthenium is 0.5 wt% or more and 2 wt% or less.

4. The catalyst according to claim 1, wherein
a supported amount of the cobalt is 10 wt% or more and 30 wt% or less.

5. The catalyst according to claim 1, wherein
the zeolite includes zeolite with pores which decomposes a carbon chain of the produced hydrocarbon, and
each pore is a mesopore having an opening diameter of 2 nm or more and 50 nm or less.

6. The catalyst according to claim 1, wherein
a ratio of silicon to aluminum (Si/Al ratio) in the zeolite is 2.5 or more and 3.5 or less.

7. A catalyst production method for producing the catalyst according to any one of claims 1 to 6, the method comprising:
a pore forming step of forming mesopores in a carrier catalyst; and
a catalyst supporting step of supporting a metal compound on the carrier catalyst,
the catalyst supporting step including a step of supporting the metal compound containing cobalt and at least one of the metal compound containing manganese and the metal compound containing ruthenium on the carrier catalyst.

8. The method for producing a catalyst according to claim 7, wherein
the catalyst supporting step includes a melt impregnation step of melt-impregnating the carrier catalyst with the metal compound containing the cobalt and at least one of the metal compound containing the manganese and the metal compound containing the ruthenium.

9. The method for producing a catalyst according to claim 8, wherein
the melt impregnation step is a step of supporting the metal compound containing the cobalt on the carrier catalyst by a melt impregnation method, and then supporting at least one of the metal compound containing the manganese and the metal compound containing the ruthenium on the carrier catalyst by a melt impregnation method.

10. The method for producing a catalyst according to claim 8, wherein
the melt impregnation step involves supporting the metal compound containing cobalt along with at least one of the metal compounds containing manganese or ruthenium in parallel using the melt impregnation method.

11. The method for producing a catalyst according to claim **7,** wherein
the catalyst supporting step includes an impregnation step of supporting the metal compound containing the cobalt on the carrier catalyst by an impregnation method, and then immersing the carrier catalyst supporting the cobalt in at least one of a solution containing the manganese and a solution containing the ruthenium to impregnate the carrier catalyst and the supported catalyst supported on the carrier catalyst with the at least one of the solution containing the manganese and the solution containing the ruthenium.

12. The method for producing a catalyst according to claim 7, wherein
the catalyst supporting step includes an impregnation step of supporting the metal compound containing the cobalt on the carrier catalyst by an impregnation method, and immersing the carrier catalyst supporting the cobalt in at least one of a solution containing the manganese and a solution containing the ruthenium to impregnate at least one of the carrier catalyst and the supported catalyst supported on the carrier catalyst with the at least one of the solution containing the manganese and the solution containing the ruthenium.

13. The method for producing a catalyst according to claim 7, wherein
the catalyst supporting step includes:
a melt impregnation step of melt-impregnating the carrier catalyst with the metal compound containing the cobalt; and
an impregnation step of immersing the carrier catalyst supporting the metal compound containing the cobalt, which is obtained in the melt impregnation step, in at least one of a solution containing the manganese and a solution containing the ruthenium to impregnate at least one of the carrier catalyst and the supported catalyst supported on the carrier catalyst with the at least one of the solution containing the manganese and the solution containing the ruthenium.

14. The method for producing a catalyst according to claim 7, wherein
in the carrier catalyst, a cation is either pre-coordinated or introduced through a cation-exchange step using an ion-exchange method, which is performed prior to the catalyst supporting step.

15. The method for producing a catalyst according to claim 14, wherein
the cation is at least one cation selected from the group consisting of lanthanum, potassium, lithium, sodium and cerium.

16. A liquid fuel production method, comprising
producing a liquid fuel including a hydrocarbon from a synthesis gas by a Fischer-Tropsch synthesis method using the catalyst according to any one of claims 1 to 6.
